# FASCICULE DE BREVET EUROPEEN

(11) **EP 3 721 224 B1**
(45) Date de publication et mention de la délivrance du brevet: **30.08.2023**
(21) Numéro de dépôt: 18808036.0
(22) Date de dépôt: 03.12.2018
(51) Int. Cl.: G01N 33/483

(54) **PLATEFORME DE NANOSTRUCTURES POUR L'INTERFAÇAGE CELLULAIRE ET PROCEDE DE FABRICATION CORRESPONDANT**
NANOSTRUKTURPLATTFORM FÜR ZELLULARE SCHNITTSTELLE UND ENTSPRECHENDES HERSTELLUNGSVERFAHREN
NANOSTRUCTURE PLATFORM FOR CELLULAR INTERFACING AND CORRESPONDING MANUFACTURING PROCESS

(30) Priorité: 05.12.2017 FR 1761651
(43) Date de publication de la demande: 14.10.2020
(73) Titulaire: Centre National de la Recherche Scientifique, 75016 Paris Cedex 16 (FR)
(72) Inventeur: LARRIEU, Guilhem, 31400 Toulouse (FR); CASANOVA, Adrien, 31400 Toulouse (FR)
(74) Mandataire: Vidon Brevets & Stratégie
(86) Numéro de dépôt international: PCT/EP2018/083305
(87) Numéro de publication internationale: WO 2019/110485

(56) Documents cités:
- WO-A2-2012/069606
- ADRIEN CASANOVA ET AL: "Probing electrical activity of single neurons based on ID nanostructures: From extra to intracellular interfacing", 2016 IEEE NANOTECHNOLOGY MATERIALS AND DEVICES CONFERENCE (NMDC), 1 octobre 2016 (2016-10-01), pages 1-2, XP055501324, DOI: 10.1109/NMDC.2016.7777156
- MORIA KWIAT ET AL: "Highly Ordered Large-Scale Neuronal Networks of Individual Cells - Toward Single Cell to 3D Nanowire Intracellular Interfaces", ACS APPLIED MATERIALS & INTERFACES, vol. 4, no. 7, 22 juin 2012 (2012-06-22), pages 3542-3549, XP055501471, US ISSN: 1944-8244, DOI: 10.1021/am300602e
- MICHELE DIPALO ET AL: "Intracellular and Extracellular Recording of Spontaneous Action Potentials in Mammalian Neurons and Cardiac Cells with 3D Plasmonic Nanoelectrodes", NANO LETTERS, vol. 17, no. 6, 24 mai 2017 (2017-05-24), pages 3932-3939, XP055501265, US ISSN: 1530-6984, DOI: 10.1021/acs.nanolett.7b01523
- LONG YANG ET AL: "Nanodevices for Cellular Interfaces and Electrophysiological Recording", ADVANCED MATERIALS, vol. 25, no. 28, 26 juillet 2013 (2013-07-26), pages 3881-3887, XP055501488, DE ISSN: 0935-9648, DOI: 10.1002/adma.201301194

## Description

### 1. Domaine

La présente technique se rapporte aux domaines des nanostructures et plus particulièrement des nanostructures à destination de captation de phénomènes biologiques. Plus particulièrement, la présente technique se rapporte à une plateforme de mesure et de stimulation de cellules de type neurales ou encore de cardiomyocytes, et plus généralement à tout type de cellules dont il est envisageable de capter une activité électrique ou électrophysiologique, pouvant être des cultures cellulaires dissociées (neurones, cellules musculaires cardiaques, ...), des cultures tissulaires d'organe ou des tranches de tissus (hippocampe, cervelet, moelle épinière, rétine, ...) ou des cultures de cellules dérivées de cellule souches.

### 2. Art Antérieur

L'étude des états électrophysiologiques des cellules excitables (neurones, cardiomyocytes) et des groupes et réseaux formés par ces cellules permet d'améliorer la compréhension des états fonctionnels et pathologiques des organes auxquels ces cellules se rapportent. L'état électrophysiologique de la cellule est traditionnellement étudié avec des sondes qui peuvent avoir accès à l'intérieur (cytoplasme) de la cellule, permettant ainsi les mesures des fluctuations de potentiels d'une ou plusieurs cellules dans un réseau de cellules. Ces sondes comprennent des « patch-clamp » avec micropipette en verre, des matrices de microélectrodes/ nanoélectrodes (MEAs / NEA), et des nano-fils (nano-sonde) à effet de champs (FET), et avec différents matériaux, tels que des réseaux de nano-fils verticaux en sillicium (Si), platine (Pt) et oxyde d'iridium (IrOx).

Des électrodes de micropipette en verre avec un procédé de « patch-clamp » ont traditionnellement été utilisées. Elles permettent de réaliser des mesures de bonne qualité, mais d'une part elles ne peuvent pas être utilisées pour un grand nombre de cellules de manière simultanée (de fait de la difficulté de mise en oeuvre de ces sondes) et d'autre part elles entraînent la mort des cellules à relativement court terme, de fait de la pénétration de la micropipette au sein de la cellule.

Plus récemment, les développements technologiques ont permis de développer des réseaux de microélectrodes planaires qui sont devenus des plateformes standards de l'étude des réponses électrophysiologiques des réseaux cellulaires sur de longues périodes (plusieurs semaines). Ces réseaux de microélectrodes présentent l'avantage de ne pas altérer l'enveloppe de des cellules et donc de ne pas entraîner la mort prématurée de celles-ci. Cependant, les réseaux de microélectrodes planaires présentent l'inconvénient de ne pas pouvoir accéder au cytoplasme cellulaire et donc de ne pas pouvoir mesurer les variations de potentiel à l'intérieur de la cellule de manière aussi précise que les électrodes à micropipette. Or la mesure des variations de potentiels est une donnée importante qu'il convient de mesurer. Le problème principal réside cependant de ces réseaux existants et de ces plateformes est la faible interaction cellule/microélectrodes qui induit des signaux (potentiel d'action) très dégradés en termes d'amplitude et donc difficilement utilisables.

Les plateformes et réseaux à base de microélectrodes ont fait l'objet de développements technologiques importants, tels que ceux présentés dans les documents US7,905,013 et WO2017127551.

Dans l'exemple de US7,905,013, une couche diélectrique sur une couche conductrice est gravée sélectivement à l'état humide, formant des trous de contact avec des parois inclinées dans la couche diélectrique et exposant des régions de la couche conductrice. Des interfaces neuronales de nano-fils IrOx sont ensuite développées à partir des régions exposées de la couche conductrice. Les interfaces neuronales à nano-fils IrOx ont chacune une section transversale comprise entre 0,5 et 10 micromètres, une hauteur moyenne comprise entre environ 10 nanomètres (nm) et environ 10 micromètres (µm) et un diamètre d'extrémité proximal moyen dans une plage d'environ 1 nm à environ 1 µm. Zhang rapporte des grappes de sondes sur des puces allant de 1 à 100 millimètres carrés. Les groupes comprennent chacun de 2 à jusqu'à 12 électrodes, situées dans un diamètre de groupe compris entre 5 et 50 micromètres, le nombre de groupes sur la puce étant compris entre 2 et 100.

Dans l'exemple de WO2017127551, un réseau de capteurs à sonde neurale est décrit. Un tel réseau comprenant un substrat avec un motif métallique sur celui-ci. Un réseau de sondes de nano-fils verticaux semi-conducteurs s'étend à distance du substrat, et les sondes sont adressées électriquement, de manière individuelle, à travers le motif métallique. Le motif métallique est isolé avec un diélectrique, et les parties de base et les tiges des nano-fils sont également de préférence isolées. Cette plateforme permet de stimuler individuellement les cellules qui prennent place sur les nano-fils.

Enfin, dans l'exemple de l'article de 2016 de Casanova A., *"Probing electrical activity of single neurone based on 1D nanostructures: from extra to intracellular interfacing",* deux types de capteurs permettant de mesurer l'activité électrique de neurones sont étudiés et comparés dans les mêmes conditions de culture, sur une même plateforme.

Bien que permettant de mesurer les potentiels électriques des cellules qui ont une activité électrique, voire de les grouper en réseaux ordonnés de cellules, ces plateformes ne permettent pas de mesurer tout à la fois les potentiels électrique et les concentrations d'ions extracellulaires. Or l'obtention de ces deux informations est préférable afin de permettre une meilleure analyse des phénomènes électrochimiques qui parcourent les réseaux de cellules.

### 3. Résumé

La présente technique a été construite sur la base de ces problèmes de l'art antérieur. Plus particulièrement, la présente technique se rapporte à une plateforme de mesure et de stimulation de cellules de type neurales ou encore de cardiomyocytes, et plus généralement à tout type de cellules dont il est envisageable de capter une activité électrique ou électrophysiologique. Plus particulièrement, une plateforme de ce type améliore les plateformes connues en ce qu'elle permet tout à la fois de mesurer des potentiels électriques et des concentrations ioniques avec des résolutions cellulaires.

Plus particulièrement, il est proposé une plateforme pour l'interfaçage cellulaire, comprenant au moins une nano-sonde à base de nano-fils comprenant chacun une extrémité conductrice destinée à être en contact avec une cellule, plateforme caractérisée en ce qu'elle comprend un ensemble de nano-capteurs, chaque nano-capteur comprenant un couple formé d'une nanosonde et d'un transistor à effet de champs, dit nano-FET espacé de ladite nanosonde de sorte que ladite nanosonde et ledit nano-FET d'un même nano-capteur interfacent avec une même cellule.

Ainsi, il est possible, avec cette plateforme, de disposer de nano-dispositifs de détection et/ou de mesure de potentiel et de concentration ionique, sur un même dispositif (la plateforme). Plus particulièrement, grâce à cette plateforme, il est possible de permet de mettre en corrélation le potentiel électrique et les courants ioniques mesurés sur ces cellules.

Selon un mode de réalisation particulier, une nano-sonde et d'un nano-FET sont espacés d'une distance comprise entre 1 et 5 micromètres, au sein d'un nano-capteur.

Ainsi, il est proposé une plateforme comprenant des nano-capteurs, par exemple répartis selon un schéma prédéterminé, qui permet de détecter et/ou mesure l'activité électrophysiologique de cellules en culture et ou en développement, sans qu'il soit nécessaire de conformer la croissance ou le développement de ces cellules. Une plateforme de ce type permet de mesurer les potentiels et les concentrations d'ion d'ensembles de cellules avec une sensibilité supérieure à celle des plateformes antérieures, de par le couplage nano-sonde et nano-FET.

Selon un mode de réalisation particulier, la plateforme comprend un ensemble de nanosondes et un ensemble de nano-FETs répartis sur ladite plateforme pour former au moins un réseau de nano-capteurs comprenant au moins un parcours prédéterminé.

Ainsi, il est proposé une plateforme qui permet tout à la fois de mesurer un potentiel électrique, à l'aide d'au moins une nano-sonde et un courant d'ion en extracellulaire avec un transistor. Dès lors, les mesures réalisables à l'aide d'une telle plateforme sont plus précises et plus fiables que celle réalisées avec les plateformes et méthodes de l'art antérieur.

Selon une caractéristique particulière, la plateforme comprend en outre, des zones hydrophiles localisées au niveau des nanosondes et dudit au moins un nano-FET.

Selon une caractéristique particulière, la plateforme comprend une pluralité de parcours prédéterminés, chaque parcours prédéterminé reliant au moins deux nanosondes dudit réseau de nanosondes.

Selon une caractéristique particulière, la plateforme comprend en outre, des zones hydrophiles localisées au niveau desdits parcours prédéterminés.

Ainsi, il est possible de favoriser la croissance des cellules sur les zones hydrophiles, et donc il est possible de conformer la croissance et le développement des cellules aux emplacements ou sont disposés les nano-dispositifs de mesure et de stimulation des cellules.

Selon une caractéristique particulière, chaque parcours prédéterminé entre deux nanosondes comprend une pluralité de nano-FETs, chaque nano-FET étant implanté à intervalles réguliers le long du parcours.

Selon une caractéristique particulière, chaque parcours prédéterminé entre deux nanosondes comprend une pluralité de nanosondes de parcours, chaque nano-sonde de parcours étant implantée à intervalles réguliers le long du parcours.

Selon un mode de réalisation particulier, ladite au moins une nano-sonde comprend entre un et neuf nano-fils.

Ainsi, la constitution de ces nanosondes, à base de un à neuf fils, permet de favoriser l'interaction entre la sonde et la cellule ; En effet, les inventeurs ont constaté qu'un nombre de fil réduit (et donc une densité de fil moins importante) sur une sonde permet à la cellule, et plus particulièrement au soma, d'épouser plus complètement la sonde, et in fine, favorise la mesure ou la stimulation. Dans un mode de réalisation particulier, une nano sonde de soma comprend quatre nano-fils.

Selon un mode de réalisation particulier, le nano-FET est un transistor à ailette de type fin-FET.

Selon un autre aspect, l'invention se rapporte également à un procédé de fabrication d'une plateforme pour l'interfaçage intracellulaire, procédé de type descendant. Selon l'invention, un tel procédé comprend, postérieurement à une étape de structuration verticale d'au moins une nano-sonde à base de nano-fils, une étape de structuration planaire d'au moins un transistor à effet de champs, dit nano-FET.

Selon une caractéristique particulière, postérieurement à l'étape de structuration planaire dudit au moins un nano-FETs, ledit procédé comprend une étape de siliciuration de platine, provoquant l'implantation d'une couche de siliciure de platine (PtSi) sur ladite au moins une nano-sonde et sur des zone source (S) et de drain (D) dudit au moins un nano-FET.

Selon une caractéristique particulière, ledit procédé comprend en outre une étape d'adjonction, sur les nano-fils de ladite au moins une nano-sonde, d'un matériau organique conducteur, tel que le PEDOT:PSS.

Selon une caractéristique particulière, postérieurement à l'étape de structuration planaire dudit au moins un nano-FETs, ledit procédé comprend une étape de fonctionnalisation de la surface de ladite plateforme définissant une pluralité de zones hydrophiles.

### 4. Dessins

D'autres caractéristiques et avantages apparaîtront plus clairement à la lecture de la description suivante d'un mode de réalisation préférentiel, donné à titre de simple exemple illustratif et non limitatif, et des dessins annexés, parmi lesquels :
- la figure 1 décrit le principe de spécialisation de surface optionnellement mis en oeuvre sur une plateforme de la présente technique ;
- la figure 2 décrit un premier mode de réalisation d'une plateforme de la présente technique ;
- la figure 3 décrit un deuxième mode de réalisation d'une plateforme de la présente technique ;
- la figure 4a est une représentation microscopique d'une nano-sonde de la présente technique ;
- la figure 4b est une représentation microscopique d'un nano-FET de la présente technique ;
- la figure 5 est une représentation microscopique d'un nano capteur comprenant une nano-sonde et un nano-FET ;
- la figure 6 est une représentation simplifiée du procédé de fabrication d'une plateforme dans un mode de réalisation particulier.

### 5. Description

### 5.1. Rappels des principes

Comme exposé précédemment, la présente technique se rapporte à une plateforme de détection, de mesure et de stimulation pour l'interfaçage intra-neuronal à l'échelle de la cellule individuelle. La plateforme repose sur des nano-dispositifs à base de nanostructures à ratio surface/volume très élevés (nano-fils (NFs), nano-ailettes (Fin) qui combinent des réseaux verticaux (clamps) et des réseaux horizontaux (nano-transistors). Ces nano dispositifs permettent de réaliser des mesures de haute sensibilité.

Ainsi, des nanostructures forment la base de nos nano dispositifs (nanosondes, nano-FETs) d'enregistrement comprenant une interface à base de « PtSi ») qui rendent possibles un enregistrement de très bonne résolution tout en restant isolé du milieu de culture. Plus particulièrement une plateforme de la présente technique comprend à la fois des nanosondes de mesure et de stimulation de cellules, à base de nano-fils verticaux et des transistors à effet de champs (nano-FETs) de mesure des concentrations d'ions traversant les cellules. La combinaison de ces deux types de nanodispositif sur la plateforme permet de réaliser des mesures de plus grande précision tout en garantissant une survie sur le « long » terme des cellules (plusieurs jours, voire semaines), de fait du caractère faiblement intrusif des nanostructures ainsi que de l'isolation entre le milieu de culture de la cellule et de la plateforme. Coupler sur la même plateforme mais surtout à une distance très réduite (de la taille d'une cellule individuelle, et ce quelle que soit la cellule en question) une nanosonde de mesures de potentiel d'action avec un nano transistor pour la mesure de concentration d'espèces ioniques ouvre de nouveaux champs d'étude en biologie. La distance séparant les deux nanodispositifs est déterminée en fonction de la cellule de destination. Cette distance peut être de quelques dizaines de nanomètre à plusieurs micromètres. Ce couple de capteurs peut être distribué sur la plateforme afin de mesurer l'activité électrique dans un réseau de cellules électriquement excitable ou des tissus ou tranches d'organes comprenant des cellules électriquement excitables. Les mêmes dispositifs peuvent simultanément exciter localement (stimuler électriquement) une cellule ou une zone de cellules puis mesurer l'activité sur cette cellule ainsi que sur l'ensemble du réseau après cette stimulation.

Selon la présente technique, un premier mode de réalisation de la plateforme se compose de couples de capteurs (nanosondes verticales et transistors à effet de champs, par exemple de type planaire, espacés par exemple de 1 à 5 µm l'un de l'autre) qui sont distribués régulièrement sur la partie utile de la surface de la plateforme afin de pouvoir accéder à une résolution spatiale du réseau de cellules et/ou de la coupe d'organe étudié, sans conformation de culture (à la différence du deuxième mode de réalisation). Il s'agit de permettre, dans ce mode de réalisation, l'étude de cellules auto-organisées. Par partie «utile de la plateforme, on entend la partie qui sert à effectuer une culture de cellules.

Selon la présente technique, un deuxième mode de réalisation de la plateforme définit un ou plusieurs réseaux de nano-dispositifs qui définissent des emplacements de positionnement et des chemins (parcours) de croissance des prolongements des cellules de sorte à former, lors de la mise en oeuvre d'un processus biologique de culture et/ou de croissance de cellules, des réseaux de cellules conformés aux réseaux de nano-dispositifs de la plateforme. Il s'agit de permettre des cultures organisées de cellules.

Quel que soit le mode de réalisation, une plateforme objet de la présente peut tout à la fois être utilisée comme plateforme de détection et/ou de mesure de potentiel et de concentration ionique, mais également comme plateforme de stimulation

Selon la présente technique, dans un autre aspect, la mise en oeuvre de ces nano-dispositifs est couplée à une fonctionnalisation de surface capable de précisément localiser les cellules et de guider la croissance des prolongements de celles-ci. Ainsi, les réseaux de dispositifs implantés sur la plateforme permettent de stimuler et d'enregistrer précisément les potentiels d'action générés par ces cellules, directement dans les somas mais aussi le long des prolongements dendritiques et axonals et cela sur plusieurs semaines.

Selon la présente technique, des transistors à nano-fils horizontaux disposés le long de ces prolongements permettent également de pouvoir comparer les enregistrements intra et extracellulaire.

Ainsi, selon la présente technique, les réseaux formés sur la plateforme sont soumis à un traitement surfacique dont la particularité est de favoriser l'implantation et la croissance des cellules à des localisations prédéfinies sur la plateforme. Plus particulièrement, la plateforme est soumise à une fonctionnalisation de surface, qui permet, en culture, de localiser les somas et de guider la croissance des prolongements neuronaux. Il est ainsi possible de stimuler et d'enregistrer précisément les potentiels d'action générés par ces neurones sur la plateforme.

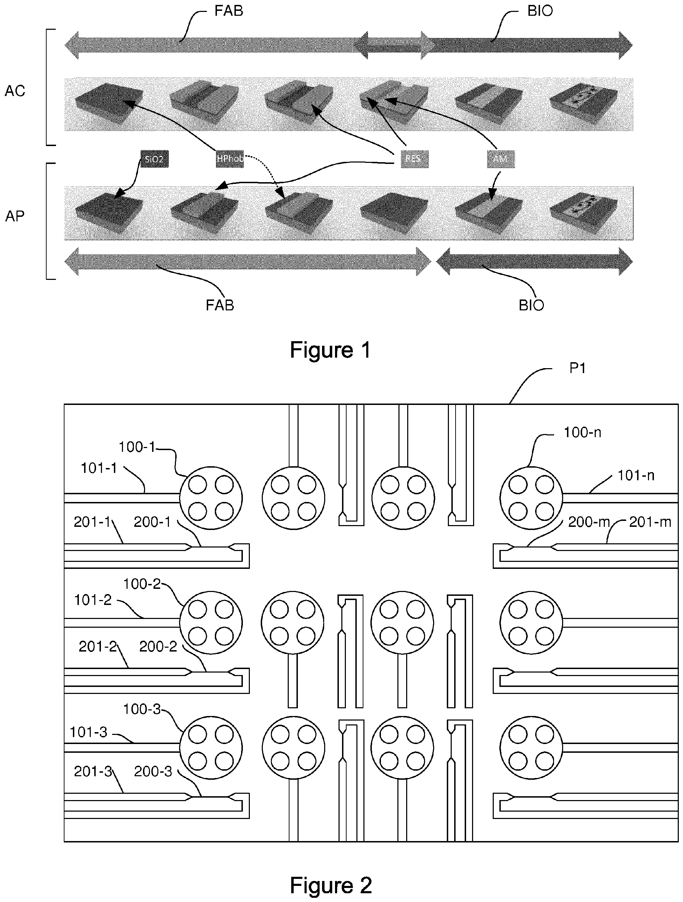

Plus particulièrement, il est optionnellement mis en oeuvre, sur la plateforme, une fonctionnalisation de surface auto alignée (S-A-F). Selon la présente technique, cette fonctionnalisation est mise en oeuvre postérieurement à la création de la plateforme. Plus particulièrement, la fonctionnalisation de surface est réalisée après la construction des réseaux de nano-dispositifs sur la plateforme. En effet, bien que la problématique de contrôle de la localisation des somas et de leurs extensions pour pouvoir coupler ces cultures organisées avec des dispositifs d'enregistrement nanométriques, ne soit pas nouvelle, les approches actuelles tendent à mixer la micro fabrication de la plateforme et le protocole biologique (consistant à déposer une couche attractive (peptides) sur la surface hydrophile pour favoriser le développement des cellules). Plus particulièrement, dans l'approche conventionnelle *(AC,* *figure 1**) (*M. Kwiat, R. Elnathan, A. Pevzner, A. Peretz, B. Barak, H. Peretz, T. Ducobni, D. Stein, L. Mittelman, U. Ashery, and F. Patolsky, "Highly Ordered Large-Scale Neuronal Networks of Individual Cells Toward Single Cell to 3D Nanowire Intracellular Interfaces," ACS Appl. Mater. Interfaces, vol. 4, no. 7, pp. 3542-3549, Jul. 2012*.),* l'étape de photolithographie permettant de définir des zones attractives et répulsives sur la puce, doit être réalisée après le traitement hydrophobe (HPhob) de l'intégralité du substrat. C'est une étape critique puisque la résine (RES) adhère très difficilement sur de telles surfaces (Hphob) et que la qualité du traitement doit être dégradée pour atteindre un niveau d'hydrophobicité compatible avec l'étape de photolithographie. Selon la présente technique (AP, Fig 1), en sus de la capacité à mesurer à la fois les concentrations d'ions et les potentiels électriques, et compte tenu des limitations de l'approche conventionnelle, une méthode de fonctionnalisation chimique complémentaire a été construite, permettant de tirer parti d'un traitement hydrophobe non dégradé, tout en dissociant complètement le protocole de microfabrication (FAB) et le protocole biologique (BIO). Contrairement à l'approche classique, pour laquelle la molécule d'adhésion (AM) se dépose seulement sur les zones ouvertes de la résine structurée par lithographie, la localisation de la molécule d'adhésion est uniquement contrôlée à l'aide du contraste hydrophile/hydrophobe de la surface.

Afin d'être le plus proche possible de la surface finale des puces formant la plateforme de capteurs (SiO2), les développements sont réalisés sur un substrat de silicium (Si) recouvert de d'environ 600 nm d'oxyde de silicium (SiO2). La structuration des motifs est effectuée par lithographie à l'aide d'un photo-répéteur i-line sur résine positive ECI (1.1 µm), permettant d'obtenir une résolution inférieure au micron, ainsi qu'une excellente précision d'alignement. Un traitement hydrophobe (HPhob) en phase gaz est ensuite réalisé sur le substrat et ne se fixe que sur les zones ouvertes de la résine. La résine est alors retirée par bains successifs d'acétone puis de « piranha » (H2O2/ H2SO4 1:1). A ce stade du procédé de fonctionnalisation, la surface de SiO2 présente des zones hydrophiles (zones précédemment protégées par la résine) et des zones hydrophobes (zones ouvertes, HPhob). Le greffage des molécules d'adhésion (AM) (de la solution d'adhésion, par exemple polylysine, polyornithine) est ensuite réalisé sélectivement sur les parties hydrophiles. Grâce à ce procédé de fonctionnalisation auto-aligné, une parfaite dissociation entre le protocole de microfabrication en salle blanche et le protocole biologique est obtenu. La solution d'adhésion n'est déposée qu'au moment de la culture et ne risque pas d'être dégradée lors du retrait de la résine. De plus, la photolithographie s'effectue avant le traitement hydrophobe de la surface ce qui permet d'éviter les problèmes liés à une mauvaise accroche de celle-ci sur le substrat hydrophobe et qui autorise l'utilisation d'un traitement hydrophobe le plus performant possible.

A partir d'une telle plateforme, il est possible de provoquer des stimulations de neurones par des agents chimiques (type simulant de gaz neurotoxiques ou induisant des dépolarisations) ou biologiques pour étudier au plus près des cellules en culture, les réactions et les processus mis en jeu et ce dans un objectif de mise au point de traitements ou de molécules adaptés à curation de maladies et notamment de maladies neuro dégénératives. Les nanosondes captent les potentiels principalement en extracellulaire. Dans quelques cas les sondes peuvent percer spontanément la membrane (très peu de cas) et mesurer le potentiel intracellulaire. On peut forcer l'entrée des sondes en ouvrant la membrane lipidique par stimulation électrique.

A noter que le potentiel électrique intracellulaire ou extracellulaire désigne la même mesure (ce ne sont pas deux potentiels différents). La différence est qu'en extracellulaire le potentiel est écranté par la membrane de la cellule et sa résolution est théoriquement plus faible. Néanmoins grâce aux nanosondes, l'affinité entre la nano-sonde et la cellule est très grande ce qui permet bien qu'étant en mesure extracellulaire d'accéder à des résolutions de signaux qui sont de très bonne qualité, bien meilleures que celles mesurées avec des microélectrodes conventionnelles. Quel que soit le mode de réalisation envisagé, la plateforme objet de l'invention offre la possibilité d'interfacer une même cellule avec deux types de nanodispositifs différents, offrant ainsi de nouvelle possibilités d'étude des cellules.

### 5.2. Description d'un premier mode de réalisation

Comme explicité, la présente technique a été mise en oeuvre pour définir une plateforme de détection, de mesure et de stimulation de cellules, et plus particulièrement de cellules électro-sensibles. La présente technique concerne une plateforme de détection et/ou de mesure et/ou stimulation à base de nanosondes et un procédé d'utilisation d'une telle plateforme pour effectuer des mesures électrophysiologiques. Dans ce premier mode de réalisation de la plateforme de la présente technique, comme illustré sur la Figure 2, des couples {nanosondes, nano-FET} sont régulièrement implantés sur la plateforme. L'objectif, dans ce mode de réalisation est d'obtenir des mesures de potentiels d'action et de concentration ionique au niveau des cellules. Chaque couple {nanosondes, nano-FET} forme un nano capteur indépendant. Au sein d'un capteur, chaque nano dispositif est adressable indépendamment : chaque nano dispositif dispose d'une ligne d'accès à partir de la périphérie de la plateforme. Dans ce mode de réalisation, des nano-FET sont des transistors à effet de champs à ailette, également appelés fin-FET. La disposition des nano-capteurs est effectuée en fonction des besoins : l'espacement entre les nano-capteurs, notamment, peut être fonction de la destination de la plateforme, c'est-à-dire d'une spécialisation de la plateforme, notamment au niveau des types de cellules à cultiver : certains types de cellules peuvent requérir des espacements plus grands ou plus petits entre les capteurs, par exemple pour éviter que deux capteurs ne soient positionnés sous une cellule unique. On note que les nanosondes ne sont pas constitués de deux nanodispositifs fabriqués indépendamment les uns des autres, mais bien de deux nanodispositifs fabriqués conjointement.

Dans ce premier mode de réalisation de la plateforme de la présente technique, comme illustré sur la figure 2, la plateforme P1, dispose d'un ensemble de nanosondes (100-1,...100-n) comprenant un à neuf nano-fils verticaux (par exemple quatre pour favoriser la descente de la cellule sur la nano sonde) directement en contact avec une ligne d'accès conductrice (101-1,... 101-n). Cet ensemble est destiné à accueillir des cellules en culture ou en observation. Chaque nano-sonde, excepté sa pointe et sa ligne d'accès conductrice (101-1...), est recouverte d'une couche isolante (non représentée). Les nanosondes (100-1,...100-n) sont en communication avec un pourtour de la plateforme via une ligne d'accès conductrice (101-1,... 101-n) dédiée, qui est connectable (connectée) électriquement à un instrument (également non représenté) pour générer ou détecter un signal électrique. La plateforme peut ainsi être utilisée pour étudier les interactions neurones-neurones, par exemple en envoyant un signal électrique dans un neurone d'un réseau neuronal via une ou plusieurs nano-sonde(s) en contact électrique avec ce neurone puis en détectant les signaux électriques d'un autre neurone du réseau via les nanosondes en contact avec cet autre neurone.

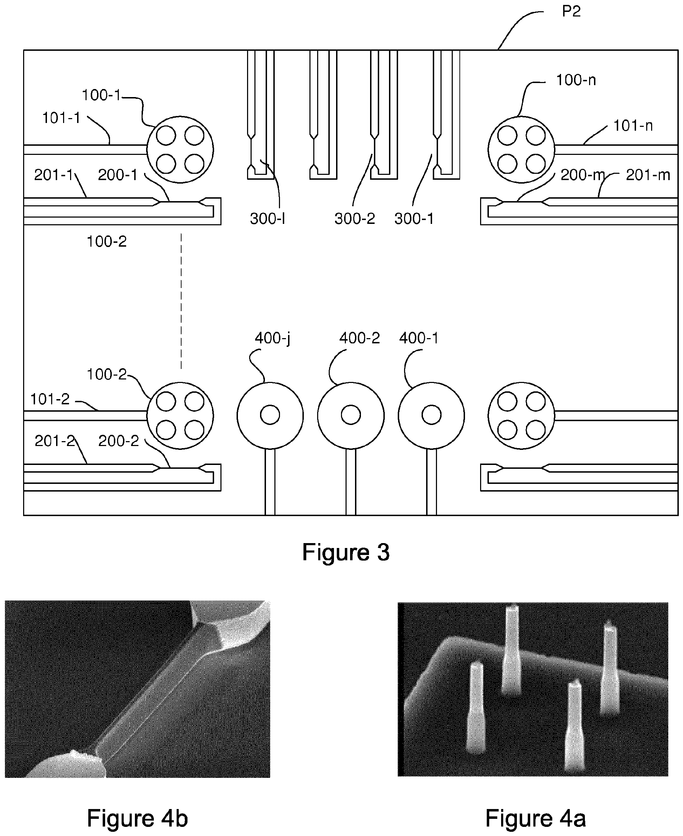

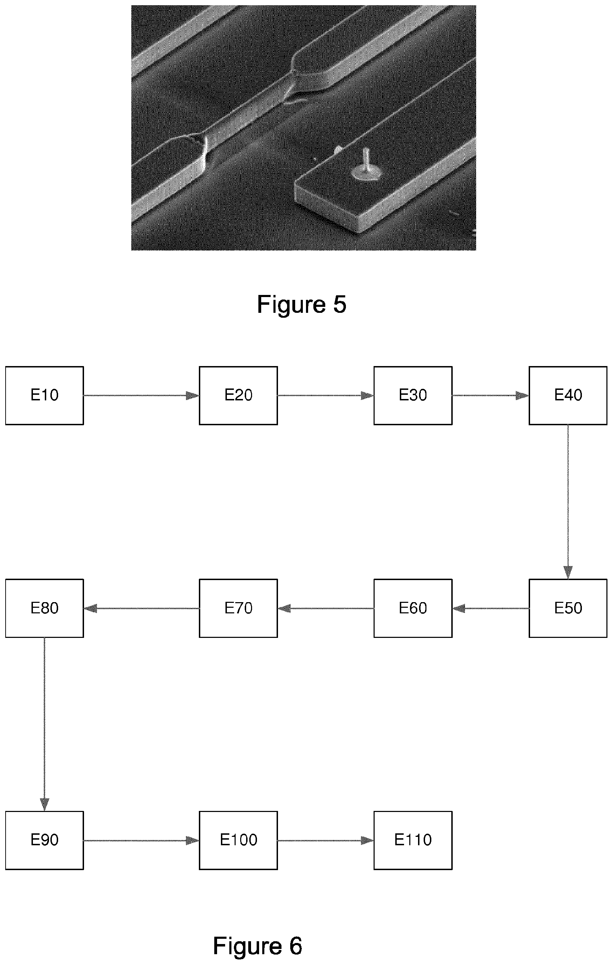

En sus de ces nanosondes, la plateforme P1 comprend des transistors à ailettes de silicium (fin-FET) permettant de détecter et mesurer les *concentrations d'ion en extracellulaire (concentration ionique). Cesfin-FETs* (200-1,...200-m) sont disposés au plus proches des nanosondes pour mesurer les concentrations ioniques résultantes. Chaque fin-FET est associé à une nano-sonde afin de former un couple {nano-sonde, fin-FET} appelé nano-capteur. Les nano-FETs (200-1,...200-m) sont en communication avec un pourtour de la plateforme via une ligne d'accès conductrice (201-1,... 201-m) dédiée, qui est connectable (connectée) électriquement à un instrument (également non représenté) pour détecter un signal électrique et plus particulièrement une concentration d'ion en extracellulaire. Ainsi, lorsqu'une activité électrique se propage dans les cellules, les nanosondes peuvent mesurer le potentiel se propageant (et notamment la vitesse de propagation de celui-ci) d'une cellule à l'autre, tout autant que les potentiels électriques de départ et d'arrivée, ainsi que les concentrations ioniques. Seuls quelques capteurs sont représentés sur la figure pour plus de lisibilité. La figure 4a est une représentation microscopique d'une nano-sonde à quatre fils verticaux de la plateforme. La figure 4b est une représentation microscopique d'un fin-FET de la plateforme. La figure 5 est une représentation microscopique d'une nano-sonde à un fil vertical associé à un fin-FET pour former un nano-capteur tel que présenté précédemment.

Dans ce premier mode de réalisation, l'objectif est de permettre une observation des comportements de groupes de cellules en croissance et/ou en culture, sans contraindre ces dernières.

### 5.3. Description d'un deuxième mode de réalisation.

Dans ce deuxième mode de réalisation de la plateforme de la présente technique, comme illustré sur la Figure 3, une cellule neuronale CN0 (ses axones ou dendrites étant marqués CN0a) est mise en contact avec la surface de la plateforme P1, qui dispose d'un réseau de nanosondes de somas (100-1,... 100-n) comprenant un à neuf nano-fils (par exemple quatre) verticaux directement en contact avec une ligne d'accès conductrice (101-1,... 101-n). Ce réseau, primaire, est appelé réseau de nanosondes de somas car il est destiné à accueillir les somas des cellules, à la différence, comme cela est exposé ci-après, de nanosondes de parcours. Chaque nano-sonde, excepté sa pointe et la ligne d'accès conductrice, est recouverte d'une couche isolante (non représentée). Les nanosondes de somas (100-1,... 100-n) sont en communication avec un pourtour de la plateforme via une ligne d'accès conductrice (101-1,... 101-n) dédiée, qui est connectable (connectée) électriquement à un instrument (également non représenté) pour générer ou détecter un signal électrique. La plateforme peut ainsi être utilisée pour étudier les interactions neurones-neurones, par exemple en envoyant un signal électrique dans un neurone d'un réseau neuronal via une ou plusieurs nano-sonde(s) en contact électrique avec ce neurone puis en détectant les signaux électriques d'un autre neurone du réseau via les nanosondes en contact avec cet autre neurone.

En sus de ces nanosondes, la plateforme P1 comprend des transistors à ailettes de silicium (nano-FET) permettant de détecter et mesurer les *concentrations d'ion en extracellulaire (concentration ionique). Ces nano-FETs* (200-1,... 200-m) sont disposés au plus proches des nanosondes de soma pour mesurer les concentrations ioniques résultantes. Des capteurs (nanosondes ou nano FETs) sont également répartis sur les chemins pour suivre la propagation Ces chemins permettent de relier les différentes nanosondes de somas (100-1,... 100-n) entre elles pour former un réseau de nano sondes de somas. L'objet de ces chemins (parcours) préétablis, comme exposé précédemment, est de guider la culture des cellules selon des paramètres prédéterminés. L'objectif de ce guidage étant de constituer un réseau de cellules reliées entre elles, de sorte à pouvoir mesurer des valeurs de potentiels et de quantité de courant ionique entre ces cellules lorsqu'elles sont positionnées (cultivées) sur le réseau de nanosondes et que les dendrites ou axones permettent la propagation de courant électrique d'une cellule à l'autre. Afin de garantir des mesures précises, les nanosondes ou nano-FETs sont positionnés le long de parcours (chemins) dont on sait, de par la spécialisation de surface de la plateforme, qu'ils seront les réceptacles des dendrites ou axones des cellules lors de leur croissance. Les nano-FETs (200-1,... 200-m) sont en communication avec un pourtour de la plateforme via une ligne d'accès conductrice (201-1,... 201-m) dédiée, qui est connectable (connectée) électriquement à un instrument (également non représenté) pour détecter un signal électrique et plus particulièrement un courant d'ion. Ainsi, lorsqu'une activité électrique se propage dans les cellules, les nanosondes peuvent mesurer le potentiel se propageant (et notamment la vitesse de propagation de celui-ci) d'une cellule à l'autre, tout autant que les potentiels électriques de départ et d'arrivée ainsi que les concentrations ioniques.

Dans au moins un mode de réalisation, tel que celui présenté en relation avec la figure 3 un parcours entre deux nano sondes de soma comprend des nano-FETs (300-1,..., 300-l) et/ou des nanosondes de parcours (400-1,..., 400-j). Ces nanosondes de parcours permettent de mesurer le potentiel électrique à différentes localisations de la dendrite ou de l'axone (seuls deux chemins sont représentés sur la figure pour plus de lisibilité).

Les nanosondes et nano-FET (transistors à ailettes de silicium) utilisés dans la présente technique peuvent être formés de n'importe quel matériau électriquement conducteur, de préférence un matériau inorganique, tel que du silicium, un métal, un semi-conducteur composé, un oxyde conducteur et un siliciure. La couche isolante revêtue sur les composants de cette plateforme est formée d'un matériau à faible cytotoxicité, tel que l'oxyde de silicium, l'oxyde d'aluminium et le nitrure de silicium. Les pointes des nano-sonde sont soit exposées (c'est-à-dire exemptes d'une couche de revêtement isolante) soit revêtues d'une couche électriquement conductrice, comme cela est explicité par la suite dans le cadre de la présentation du procédé de fabrication de ladite plateforme, selon une approche descendante. La couche conductrice électrique est également formée d'un matériau à faible cytotoxicité, tel que l'or, l'argent et le platine, un alliage de ces métaux ou un matériau organique conducteur (par exemple pedot :pss).

### 5.4. Détails du procédé de fabrication de plateforme en co-intégration

Une plateforme de mesure à base de nano-fils, telle que présentée précédemment, est obtenue à partir d'un substrat 10.16cm (4 pouces) de silicium sur isolant (SOI) (Si actif 4µm/Oxyde enterré 1µm/substrat 400µm). Une approche (descendante) « *top-down »* est utilisée avec un procédé de fabrication en photolithographie UV à l'aide d'un photorépéteur i-line suivi d'une gravure plasma RIE, afin de réduire les coûts de fabrication et de faciliter une fabrication à grande échelle. Pour augmenter la reproductibilité du procédé sur l'ensemble du substrat, une couche anti-réflective (BARC) est utilisée sous la résine photosensible.

Plus particulièrement, en relation avec la figure 6, le procédé de fabrication comprend les étapes suivantes, qui sont réalisées sur une base comprenant un substrat sur lequel est déposée une couche de 1µm de dioxyde de silicium (SiO₂), elle-même recouverte d'une couche de silicium (Si) monocristallin de 4µm :
- fabrication (E10) de nano-Fils verticaux : structuration verticale de la plateforme ;
   ∘ cette fabrication se base sur une technique de Photolithographie, comprenant un dépôt local d'un motif de résine qui sert de masque de protection (nano-plot résistant de 500 nm de diamètre), en utilisant une résine organique conventionnelle (par exemple de type ex : ECI 3012) ; et
   ∘ gravure RIE (gravure ionique réactive profonde) de 3,5 µm : gravure sèche par bombardement d'ions (issus d'un plasma); cette technique présente l'avantage d'une forte anisotropie de la gravure: la frontière entre les zones gravées et non gravées sera la plupart du temps rectiligne et verticale ; la résine restante est retirée par gravure chimique (résine issue de la sous étape précédente) ;
   ∘ à l'issue de cette première étape, on dispose sur la plateforme de nano fils verticaux, formant des groupes constitués de 1 à 4 nano-fils et répartis sur la plateforme pour former un embryon de réseau, qui est retravaillé dans les étapes suivantes afin de leur adjoindre la propriétés attendues ;
- création (E20) des nano-FET et des lignes d'accès : structuration planaire de la plateforme :
   ∘ dépôt d'une photo-résine épaisse (5 µm) pour protéger et ne pas détériorer les nano-fil précédemment obtenus ;
   ∘ photolithographie pour définir la zone à masquer selon un procédé identique à celui de l'étape précédente pour dessiner les nano-FETs (canaux et lignes d'accès), ainsi que les lignes d'accès des nanosondes ;
   ∘ gravure jusqu'à atteindre l'oxyde enterré (ou Burried Oxide - BOx) du SOI : le silicium est creusé sur une profondeur d'environ 0,5µm.
   ∘ à l'issue de cette deuxième étape, on dispose sur la plateforme de nano fils verticaux et d'embryon de nano-FET, rattachés par les lignes d'accès à des contacts électriques au bord de la plateforme, qui comme les nano-fils, sont répartis sur la plateforme pour compléter le ou les réseaux de groupes de nano-fils ;
- amincissement (E30) conjoint des volumes des nano-fils et des ailettes des nano-FETs par oxydation sacrificielle :
   ∘ une oxydation humide à environ 850 °C pendant une durée de 60 mn pleine plaque est réalisée; cette opération permet de préparer la réduction de diamètre des nano-fils d'environ 500 nm à environ 200 nm et de préparer l'amincissement du canal du nano-FET à environ 20nm ;
   ∘ cette opération permet également d'éliminer les défauts de surface induits par la gravure plasma et d'améliorer l'anisotropie de la nano-sonde ;
   ∘ le canal de chaque nano-FET est ensuite protégé par de la résine par photolithographie ;
   ∘ puis une gravure humide Buffer HF (5%) est effectuée : elle permet de retirer l'oxyde sur l'ensemble des surfaces, à l'exception de celles des canaux des nano-FET car la résine n'est pas attaquée: chaque canal est, de reste, recouvert d'oxyde (SiO₂).
- dopage (E40) de type P des zones source (S) et drain (D) des nano-FET, des nano-Fil et des lignes d'accès :
   ∘ cette étape consiste en une implantation de Bore à 10²⁰atm/cm3 sur l'ensemble de la plateforme à l'exception des canaux des nano-FET, qui restent protégés par la résine de l'étape précédente ;
   ∘ le canal n'est pas dopé afin de conserver la sensibilité de celui-ci.
- retrait de la résine de masquage du canal des nano FET et recuit d'activation du dopant :
   ∘ La résine est retirée du canal et le dopant est activé par température (environ 1000°c pendant environ 30 minutes) ;
   ∘ cette phase de recuit active électroniquement le dopant dans le silicium ;
   ∘ à l'issue de cette étape, on dispose d'une plateforme comprenant les nano-Fils verticaux, les zones S et D des nano-FET et les lignes d'accès en Si dopé : seuls les canaux restent recouverts d'oxyde.
- siliciuration (E50) du platine :
   ∘ pour créer des zones S et D (des nano FET) faiblement résistives et améliorer l'interface entre la nano-sonde et le milieu liquide, une étape de dépôt isotrope de platine pleine plaque est effectuée ;
   ∘ un recuit d'activation (500°C pdt 3 mn) permet ensuite de créer l'alliage *PtSi* ; L'avantage ici est que le *PtSi* ne se crée que lorsque le Pt est en contact direct avec le *Si* et non du *SiO₂* : la technique employée permet donc de ne pas avoir de *PtSi* sur le BOx (constitué de SiO₂), ni sur le canal des nano-FETs, permettant ainsi de conserver la sensibilité de mesure de ceux-ci.

On note ici un avantage du procédé de fabrication tel que proposé : il n'est pas nécessaire de réaliser une étape de lithogravure pour l'implantation sur le métal : on n'a pas besoin de couvrir le canal par de la résine car il est déjà protégé par de l'oxyde de Si. Il ne s'agit cependant pas du seul avantage : cette étape de siliciuration du platine permet d'augmenter fortement l'intégration des nano-dispositifs, tout en autorisant une forte biocompatibilité avec le milieu vivant et ce grâce au recuit d'activation du platine sur le *Si.*
- gravure (E60) sélective du Pt par rapport au *PtSi* à l'eau régale (mélange chimique de HCl : HNO₃ : EDI qui permet de graver seulement le métal : le silicium n'est pas attaqué) :
   ∘ il s'agit d'une étape de gravure chimique sélective qui permet de graver le Pt non transformé (issu de l'étape précédente) sans attaquer le *PtSi* : le Pt (non transformé) est donc ôté du BOX et des canaux des nano-FET ;
   ∘ L'intérêt du PtSi est double : du point de vue des nanosondes il ne s'oxyde pas (en comparaison du Si seul) et permet de conserver une faible impédance d'interface électrolyte/sonde dans le temps ; du point de vue des nano-FETs : il délivre une faible résistance de contact surface/volume améliorant la sensibilité des nano-FETs.
- métallisation (E70) à l'aluminium (Alu) permettant réduire la résistance des lignes d'accès :
   ∘ un dépôt conforme sur l'ensemble de la plateforme d'Alu (500 nm) suivi d'une photolithographie et d'une gravure chimique de l'alu non protégé par la résine *« etch-back »* sont ensuite mis en oeuvre pour métalliser les lignes d'accès ;
   ∘ l'alu n'est conservé que sur les lignes d'accès.
- isolation (E80) de la plateforme par rapport au milieu :
   ∘ Un oxyde (oxyde d'isolation) est ensuite déposé de manière conforme pour isoler les nano fils et les nano-FETs du (futur) milieu de culture : l'oxyde employé peut être du SiO2, de AL2O3 ou du HfO2, ou un diélectrique spécifique ;
- ouverture (E90) du sommet des nano-fils, des contacts extérieurs et du canal des nano-FET (structure coeur-coquille) :
   ∘ une étape de photolithographie est utilisée à l'aide d'une résine épaisse (5 µm) pour graver l'oxyde (d'isolation), précédemment déposé, au niveau des contacts extérieurs (des lignes d'accès) et du canal du nano-FET (entre la source et le drain) ;
   ∘ la résine restante est ensuite réduite en épaisseur par gravure plasma O2 pour libérer le haut de la nano-sonde ;
   ∘ la hauteur de résine peut varier entre 1 et 3 µm en fonction de la dimension du contact sensible désirée pour la nano-sonde.
- gravure (E100) chimique de l'oxyde ouvert :
   ∘ Les zones ouvertes de la résine sont gravées chimiquement à l'aide d'une solution de HF tamponnée (buffer HF) de (5%) ; cette gravure permet de libérer le sommet de la nano-sonde, de désoxyder les contacts extérieurs et de réduire l'épaisseur d'oxyde sur le canal pour améliorer la détection et/ou la mesure ;
   ∘ la résine est ensuite éliminée ;
   ∘ à l'issue de cette étape, on dispose d'une plateforme comprenant un réseau de nano-fils formant des groupes de nano-fils (un groupe comprenant de un à quatre nano-fils), des nano-FETs disposés à proximité des nanosondes ; et de lignes d'accès à l'ensemble de ces composants, à partir de la périphérie de la plateforme, et ce quelle que soit la cartographie de celle-ci.

En l'état, à l'issue de cette étape de gravure chimique d'oxyde, cette plateforme, fabriquée selon le procédé précédemment exposé, permet de résoudre la problématique liée à la mesure *conjointe,* à l'aide d'une même plateforme, d'un potentiel électrique, à l'aide d'au moins une nano-sonde et de concentration d'ion en extracellulaire avec un ou plusieurs transistors (nano-FET). L'ensemble de ces étapes permet d'obtenir un dispositif, ou une plateforme, en une seule passe, ce qui n'est pas permis par les méthodes de fabrication de l'art antérieur. Cependant, afin de permettre ultérieurement, des expérimentations sur des cultures neuronales avec une croissance dirigée des cellules sur la plateforme, les inventeurs ont mis en oeuvre une étape supplémentaire de spécialisation de la surface de la plateforme, cette étape supplémentaire permettant de contraindre la culture et la croissance des cellules à des localisations prédéterminées, comme cela a été présenté précédemment. Plus particulièrement, selon la présente technique, le procédé de fabrication comprend en outre une étape de fonctionnalisation (E110) chimique comprenant une photolithogravure de définition de motifs d'accroche des cellules et plus spécifiquement de motifs hydrophiles.

Ces motifs sont réalisés au niveau des zones spécifiques de plateforme sur lesquelles on souhaite favoriser la croissance des cellules. Des zones hydrophiles de plus grandes surfaces sont créées au niveau des nanosondes, aux endroits où on souhaite favoriser l'implantation de soma. Des zones hydrophiles sont également créées dans le prolongement des nanosondes, afin de permettre aux prolongements de cellules (axones, dendrites si nécessaires) afin de diriger la croissance de ces prolongements vers d'autres nanosondes, sur lesquelles d'autres somas seront fixés.

### 5.5. divers

Toutes les caractéristiques décrites peuvent être combinées et ce dans n'importe quelle combinaison.

De manière complémentaire aux aspects précédemment décrit, la plateforme objet de la présente peut être mise en oeuvre de différentes manières. Un avantage de cette plateforme, apporté par le fait que chaque nano-dispositif est adressable, est qu'elle permet de réaliser une localisation des cellules déposées (et/ou mise en développement) à des endroits très précis de la plateforme, ce qui n'est pas permis par l'art antérieur. Par ailleurs, l'utilisation de la plateforme n'est pas limitée à des cellules de type neurone, mais peut être mise en oeuvre pour tout type de cellules, avec une modification de la distance séparant les composantes des nanosondes. Les nanosondes peuvent être utilisées pour mesurer un potentiel d'une part et transmettre une impulsion électrique aux cellules, d'autre part. Cette transmission d'impulsion peut être utilisée pour ouvrir localement la paroi de la cellule stimulée, de manière précise et réversible. La transmission d'impulsion peut également être utilisée, dans certaines conditions, pour provoquer une excitation de la cellule.

## Revendications

1. Plateforme pour l'interfaçage cellulaire (P1, P2), comprenant au moins une nanosonde (100-1,...100-n) à base de nano-fils comprenant chacun une extrémité conductrice destinée à être en contact avec une cellule, plateforme **caractérisée en ce qu'**elle comprend un ensemble de nano-capteurs, chaque nano-capteur comprenant un couple formé d'une nanosonde (100-1) et d'un transistor à effet de champs, dit nano-FET (200-1) espacé de la nanosonde (100-1) de sorte que ladite nanosonde et ledit nano-FET d'un même nano-capteur interfacent avec une même cellule.

2. Plateforme selon la revendication 1, **caractérisée en ce qu'**au sein d'un nano-capteur, une nano-sonde (100-1) et un nano-FET (200-1) sont espacés d'une distance comprise entre 1 et 5 micromètres.

3. Plateforme selon la revendication 1, **caractérisée en ce qu'**elle comprend un ensemble de nanosondes et un ensemble de nano-FETs répartis sur ladite plateforme pour former au moins un réseau de nano-capteurs comprenant au moins un parcours prédéterminé.

4. Plateforme selon la revendication 1, **caractérisée en ce qu'**elle comprend en outre, des zones hydrophiles localisées au niveau des nanosondes et dudit au moins un nano-FET.

5. Plateforme selon la revendication 3, **caractérisée en ce qu'**elle comprend une pluralité de parcours prédéterminés, chaque parcours prédéterminé reliant au moins deux nanosondes dudit réseau de nanosondes.

6. Plateforme selon la revendication 5, **caractérisée en ce qu'**elle comprend en outre, des zones hydrophiles localisées au niveau desdits parcours prédéterminés.

7. Plateforme selon la revendication 5, **caractérisée en ce que** chaque parcours prédéterminé entre deux nanosondes comprend une pluralité de nano-FETs, chaque nano-FET étant implanté à intervalles réguliers le long du parcours.

8. Plateforme selon la revendication 5, **caractérisée en ce que** chaque parcours prédéterminé entre deux nanosondes comprend une pluralité de nanosondes de parcours, chaque nano-sonde de parcours étant implantée à intervalles réguliers le long du parcours.

9. Plateforme selon l'une des revendications 1 à 8, **caractérisé en ce que** ladite au moins une nano-sonde comprend entre un et neuf nano-fils.

10. Plateforme selon l'une des revendications 1 à 9, **caractérisé en ce que** le nano-FET est un transistor à ailette de type fin-FET.

11. Procédé de fabrication d'une plateforme pour l'interfaçage intracellulaire selon l'une des revendications 1 à 10, le procédé de fabrication comprend les étapes suivantes, qui sont réalisées sur une base comprenant un substrat de silicium sur isolant (SOI) et selon une approche de type descendante, **caractérisé en ce qu'**il comprend, postérieurement à une étape de structuration verticale d'au moins une nano-sonde à base de nano-fils, une étape de structuration planaire d'au moins un transistor à effet de champs, dit nano-FET.

12. Procédé de fabrication d'une plateforme pour l'interfaçage intracellulaire, selon la revendication 11, **caractérisé en ce que** postérieurement à l'étape de structuration planaire dudit au moins un nano-FET, ledit procédé comprend une étape de siliciuration de platine, provoquant l'implantation d'une couche de siliciure de platine (PtSi) sur ladite au moins une nano-sonde et sur des zone source (S) et de drain (D) dudit au moins un nano-FET.

13. Procédé de fabrication d'une plateforme pour l'interfaçage intracellulaire, selon la revendication 11, **caractérisé en ce qu'**il comprend en outre une étape d'adjonction, sur les nano-fils de ladite au moins une nano-sonde, d'un matériau organique conducteur, tel que le PEDOT:PSS.

14. Procédé de fabrication d'une plateforme pour l'interfaçage intracellulaire, selon la revendication 11, **caractérisé en ce que** postérieurement à l'étape de structuration planaire dudit au moins un nano-FETs, ledit procédé comprend une étape de fonctionnalisation de la surface de ladite plateforme définissant une pluralité de zones hydrophiles.

## Patentansprüche

1. Plattform für Zell-Interfacing (P1, P2), die mindestens eine Nanosonde (100-1,.... 100-n) auf der Basis von Nanodrähten umfasst, die jeweils ein leitendes Ende umfassen, das dazu bestimmt ist, mit einer Zelle in Kontakt zu sein, wobei die Plattform **dadurch gekennzeichnet ist, dass** sie einen Satz von Nanosensoren umfasst, wobei jeder Nanosensor ein Paar umfasst, das aus einer Nanosonde (100-1) und einem Feldeffekttransistor, einem so genannten Nano-FET (200-1), gebildet wird, der von der Nanosonde (100-1) so beabstandet ist, dass die Nanosonde und der Nano-FET eines gleichen Nanosensors mit einer gleichen Zelle zusammenwirken.

2. Plattform nach Anspruch 1, **dadurch gekennzeichnet, dass** innerhalb eines Nanosensors eine Nanosonde (100-1) und ein Nano-FET (200-1) in einem Abstand von zwischen 1 und 5 Mikrometern liegen.

3. Plattform nach Anspruch 1, **dadurch gekennzeichnet, dass** sie einen Satz von Nanosonden und einen Satz von Nano-FETs umfasst, die über die Plattform verteilt sind, um mindestens ein Netzwerk von Nanosensoren zu bilden, das mindestens einen vorbestimmten Pfad umfasst.

4. Plattform nach Anspruch 1, **dadurch gekennzeichnet, dass** sie außerdem hydrophile Bereiche umfasst, die auf der Ebene der Nanosonden und des mindestens einen Nano-FET lokalisiert sind.

5. Plattform nach Anspruch 3, **dadurch gekennzeichnet, dass** sie eine Vielzahl von vorbestimmten Pfaden umfasst, wobei jeder vorbestimmte Pfad mindestens zwei Nanosonden des Nanosondennetzwerks verbindet.

6. Plattform nach Anspruch 5, **dadurch gekennzeichnet, dass** sie außerdem hydrophile Zonen umfasst, die an den vorbestimmten Pfaden lokalisiert sind.

7. Plattform nach Anspruch 5, **dadurch gekennzeichnet, dass** jeder vorbestimmte Pfad zwischen zwei Nanosonden eine Vielzahl von Nano-FETs umfasst, wobei jeder Nano-FET in regelmäßigen Abständen entlang des Pfades implantiert ist.

8. Plattform nach Anspruch 5, **dadurch gekennzeichnet, dass** jeder vorbestimmte Pfad zwischen zwei Nanosonden eine Vielzahl von Pfad-Nanosonden umfasst, wobei jede Pfad-Nanosonde in regelmäßigen Abständen entlang des Pfades implantiert ist.

9. Plattform nach einem der Ansprüche 1 bis 8, **dadurch gekennzeichnet, dass** die mindestens eine Nanosonde zwischen einem und neun Nanodrähten umfasst.

10. Plattform nach einem der Ansprüche 1 bis 9, **dadurch gekennzeichnet, dass** der Nano-FET ein Transistor mit Flossen vom Fin-FET-Typ ist.

11. Verfahren zur Herstellung einer Plattform für intrazelluläres Interfacing nach einem der Ansprüche 1 bis 10, wobei das Herstellungsverfahren die folgenden Schritte umfasst, die auf einer Basis, die ein Substrat aus Silizium auf Isolator (SOI) umfasst, und nach einem Top-Down-Ansatz ausgeführt werden, **dadurch gekennzeichnet, dass** es nach einem Schritt der vertikalen Strukturierung von mindestens einer Nanosonde auf Basis von Nanodrähten einen Schritt der planaren Strukturierung von mindestens einem Feldeffekttransistor, eines so genannten Nano-FETs, umfasst.

12. Verfahren zur Herstellung einer Plattform für intrazelluläres Interfacing nach Anspruch 11, **dadurch gekennzeichnet, dass** das Verfahren nach dem Schritt der planaren Strukturierung des mindestens einen Nano-FET einen Schritt der Platinsilizidierung umfasst, wodurch die Implantation von einer Schicht aus Platinsilizid (PtSi) auf die mindestens eine Nanosonde und auf einem Source- (S) und Drain- (D) Bereich des mindestens einen Nano-FET bewirkt wird.

13. Verfahren zur Herstellung einer Plattform für intrazelluläres Interfacing nach Anspruch 11, **dadurch gekennzeichnet, dass** es ferner einen Schritt der Hinzufügung eines leitfähigen organischen Materials wie PEDOT:PSS auf den Nanodrähten der mindestens einen Nanosonde umfasst.

14. Verfahren zur Herstellung einer Plattform für intrazelluläres Interfacing nach Anspruch 11, **dadurch gekennzeichnet, dass** das Verfahren nach dem Schritt der planaren Strukturierung des mindestens einen Nano-FETs einen Schritt der Funktionalisierung der Oberfläche der Plattform umfasst, die eine Vielzahl von hydrophilen Bereichen definiert.

## Claims

1. Platform for cellular interfacing (P1, P2) comprising at least one nano-probe (100-1,...100-n) based on nano-wires each comprising a conductive extremity intended to be in contact with a cell, said platform being **characterized in that** it comprises a set of nano-sensors, each nano-sensor comprising a pair formed by a nano-probe (100-1) and a field-effect transistor called a nano-FET (200-1) spaced from the nano-probe (100-1) so that said nano-probe and said nano-FET of a same nano-sensor interface with a same cell.

2. Platform according to claim 1, **characterized in that** within a nano-sensor, a nano-probe (100-1) and a nano-FET (200-1) spaced out by a distance of 1 to 5 micrometers.

3. Platform according to claim 1, **characterized in that** it comprises a set of nano-probes and a set of nano-FETs distributed on said platform to form at least one array of nano-sensors comprising at least one predetermined route.

4. Platform according to claim 1, **characterized in that** it further comprises hydrophilic zones localized at the level of the nano-probes and of said at least one nano-FET.

5. Platform according to claim 3, **characterized in that** it comprises a plurality of predetermined routes, each predetermined route connecting at least two nano-probes of said array of nano-probes.

6. Platform according to claim 5, **characterized in that** it furthermore comprises hydrophilic zones localized at the level of said predetermined routes.

7. Platform according to claim 5, **characterized in that** each predetermined route between two nano-probes comprises a plurality of nano-FETs, each nano-FET being implanted at regular intervals along the route.

8. Platform according to claim 5, **characterized in that** each predetermined route between two nano-probes comprises a plurality of route nano-probes, each route nano-probe being implanted at regular intervals along the route.

9. Platform according to one of the claims 1 to 8, **characterized in that** said at least one nano-probe comprises between one and nine nano-wires.

10. Platform according to one of the claims 1 to 9, **characterized in that** the nano-FET is a fin-FET type of finned transistor.

11. Method for fabricating a platform for intracellular interfacing according to one of claims 1 to 10, said method comprises the following steps, which are performed on a base comprising silicon-on-insulator (SOI) substrate, and using a top-down approach, **characterized in that**, subsequently to a step of vertical structuring of at least one nano-wire-based nano-probe, a step of planar structuring of at least one field-effect transistor called a nano-FET.

12. Method for fabricating a platform for intracellular interfacing according to claim 11, **characterized in that**, subsequently to the step of planar structuring of said at least one nano-FET, said method comprises a step of platinum silicidation prompting the implantation of a layer of platinum silicide (PtSi) on said at least one nano-probe and on the source zone (S) and drain zone (D) of said at least one nano-FET.

13. Method for fabricating a platform for intracellular interfacing according to claim 11, **characterized in that** it further comprises a step of adding a conductive organic material such as PEDOT:PSS, to the nanowires of said at least one nanoprobe.

14. Method for fabricating a platform for intracellular interfacing according to claim 11, **characterized in that** subsequently to the step of planar structuring of said at least one nano-FET, the method comprises a step of functionalization of the surface of said platform defining a plurality of hydrophilic zones.
